Europäisches Patentamt

European Patent Office

Office européen des brevets

(19)

(11) Publication number : **0 475 702 A1**

## (12) EUROPEAN PATENT APPLICATION

(21) Application number : **91308212.9**

(22) Date of filing : **09.09.91**

(51) Int. Cl.⁵ : **A61F 13/62**

(30) Priority : **10.09.90 NZ 235229**

(43) Date of publication of application :
**18.03.92 Bulletin 92/12**

(84) Designated Contracting States :
**AT BE CH DE DK ES FR GR IT LI LU NL SE**

(71) Applicant : **Rutledge, Guy**
**28 Geraldine Road**
**Strand-on-the-Green, London W4 3PA (GB)**

(72) Inventor : **Neesham, Douglas James**
**10,Earle Street**
**Parnell, Auckland (NZ)**

(74) Representative : **Carter, Gerald et al**
**S. Jones-Robinson & Co., 30 St. Catherine**
**Street**
**Gloucester GL1 2BX (GB)**

㉜ **Nappy.**

㉗   A washable reusable nappy comprises a fibrous pad sandwiched between layers (16, 17) of woven cotton, shaped and sewn to provide a form which can readily be correctly fastened on a baby, and having releasable hook-and-loop fabric fasteners (24, 25) permanently attached to fastening tabs (11, 12, 13, 14). Two of the fastening tabs (11, 12) carrying the hook fasteners (24) have pockets which can be inverted so as to cover the fasteners and protect them from contact with lint or other fabric articles during washing.

EP 0 475 702 A1

FIG 1

The present invention provides a reusable, washable nappy for babies.

It has hitherto been known to provide a reusable nappy comprising a single square sheet of fabric, which can be folded in a number of ways to provide a nappy having several layers of cloth around the crotch region, and long flaps which can wrap around the baby and be pinned in place to hold the nappy on. When washing is required, the nappy can be unfolded to facilitate rapid washing and drying.

Such nappies have been found disadvantageous in a number of ways. They require a modicum of skill to fold properly, the requisite pins are inconvenient to use, and they are relatively loose fitting because of the lack of elastic at the hems.

An alternative to such nappies has been a disposable nappy, formed from plastics and wood pulp, having a thin plastics outer surface, a pad of bleached wood pulp or other fibre in the crotch region, and an inner sheet of thin gauze of paper-like material, usually also formed from wood pulp fibres, stabilised in a sheet with speckles of wax or plastics to bind the fibres together. This assemblage has strands of elastic built into the waist and leg hems, and is shaped with a curvature and extended tabs to easily fit onto a baby. The nappy is fastened in place with adhesive strips or the like.

Principal disadvantages with such nappies have been their expense, and the fact that they can only practically be used once, because the wood pulp readily disintegrates if washed and the adhesive is only effective for a single use. The adhesive tabs have been found particularly disadvantageous, in that if the adhesive is weak the nappy is likely to fall off in use, and if the adhesive is strong it tends to tear the plastics outer surface to which it adheres, if an attempt is made to disengage it. This can result in the nappy being unintentionally torn when being checked or adjusted, as if often required.

Disposable nappies also pose a considerable problem in their ultimate destruction, in that when thrown away they comprise a substantial mass of organic matter, sealed against decomposition in a plastic wrapper. The large number of nappies required for a baby means that a large volume of substantially non-decomposing waste is produced by a population using such nappies, with no effective means for destroying it.

The present invention sets out to provide an improved form of washable, reusable nappy.

According to the invention there is provided a washable nappy comprising two sheets of fabric sewn together with a pad of fibrous material sandwiched between them, and provided with reusable fastening elements.

The nappy is preferably of generally oblong shape and has four corners extended to provide fastening tabs, the reusable fastening elements being provided on said tabs.

Said fastening elements may comprise complementary strips of press fastening material, such as hook-and-loop material. Each strip of hook material may be mounted on a tab projecting outwardly from the general body of the nappy, said tab being formed of flexible material and provided with a pocket thereon, said strip of hook material being mounted on an outer wall of said pocket, whereby said pocket may be inverted so that the strip lies within the pocket.

In any of the above arrangements one or both of said two sheets of fabric may comprise woven fabric. The sheets are preferably formed at least in part from cotton.

A number of elastic elements may be fastened to the nappy and adapted to bunch or gather portions of the nappy in regions between said fastening elements.

Said sheets are preferably sewn together around and across said pad of fibrous material.

The following is a description of one preferred form of the present invention, given by way of example only, with reference to the accompanying drawings in which:

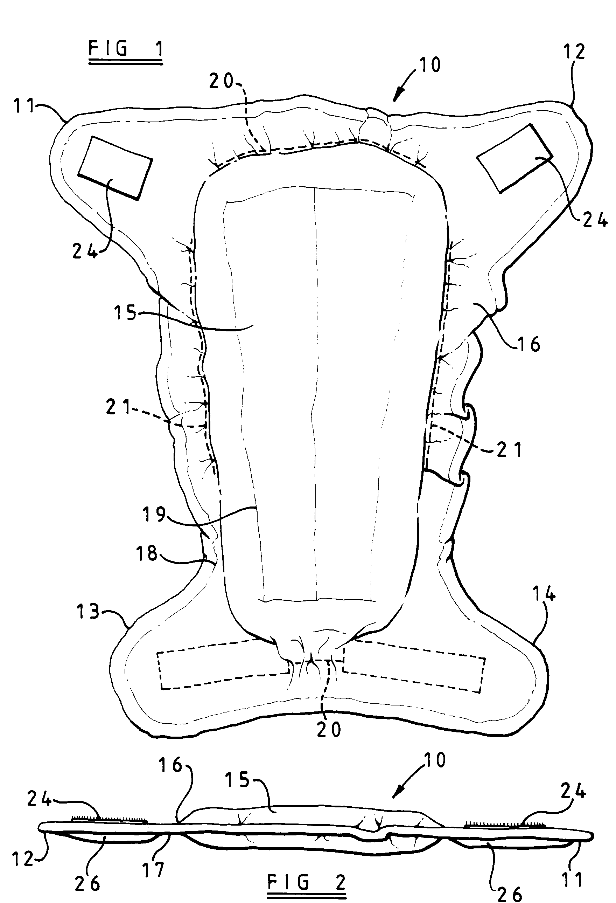

Figure 1 shows the inside surface of one embodiment of nappy in accordance with the invention;

Figure 2 shows the embodiment of Figure 1 in end view;

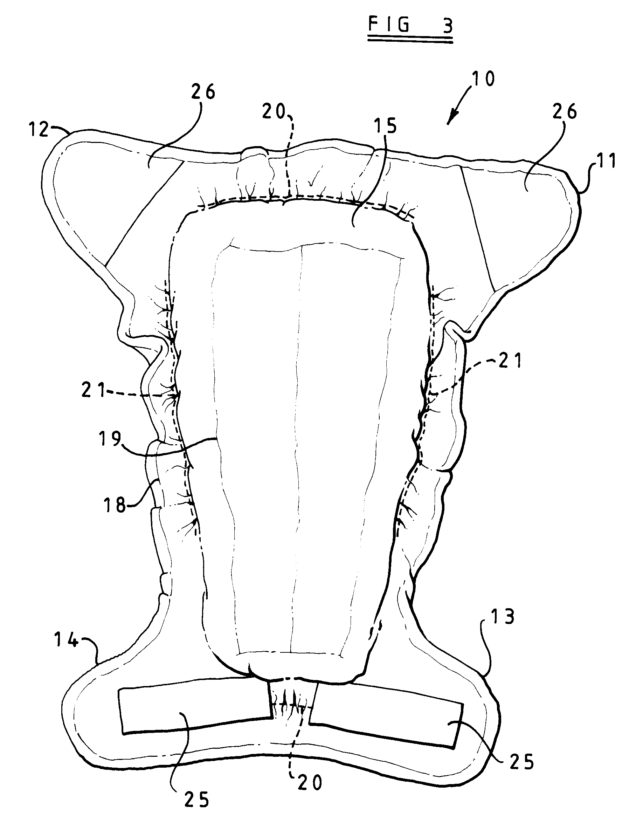

Figure 3 shows the outside surface of the embodiment of Figure 1; and

Figure 4 shows the embodiment of Figure 1 in perspective view, partly folded as in use, and with one fastening tab pocket inverted.

As illustrated in the accompanying drawings, the preferred example of the present invention comprises a nappy 10, constructed from fabric such as cotton, which is able to be washed and reused a number of times.

The nappy 10 is cut and sewn to a shape permitting relatively easy fitting onto a baby. Preferably a range of sizes can be produced, to suit different sizes of baby. Four tabs 11, 12, 13 and 14 are provided at the corners as shown, and a pad 15 is provided in the centre. The pad 15 is preferably comprised of a filling of woven or non-woven cotton fibre or other fibrous absorbent material between the inner 16 and outer 17 layer of the nappy 10. The layers 16 and 17 preferably both comprise sheets of woven cotton or cotton mix fabric, sewn together at the hem 18 and around the edges of the pad 15. Additional lines of stitching 19 may be provided across the pad 15, to fix the filling in place between the layers 16 and 17.

Strips of elastic 20 and 21 may be provided, also sewn between the layers 16 and 17, to constrict the waist and leg apertures 22 and 23 in use, thereby providing a tight but comfortable fit around the baby.

The tabs 11, 12, 13 and 14 are provided with fasteners 24 and 25, preferably comprising strips of hook-and-loop material sold under the Trade Mark "Velcro", or similar press fasteners. Such fasteners generally comprise pairs of pieces of engageable material, one piece 24 having nylon hooks thereon, and the other 25 having loops with which the hooks engage. A difficulty hitherto encountered with such fasteners when used on clothing has been that the hooks tend to snag on lint and other fabrics during washing and/or machine drying. Eventually the hooks can become substantially entirely entangled, and lose their ability to fasten, and furthermore, there is a significant risk of damage being done to other clothing by the fasteners, particularly delicate fabrics such as silk. While it is unusual for nappies and silks to be washed together, it would nonetheless be desirable to avoid such problems where possible.

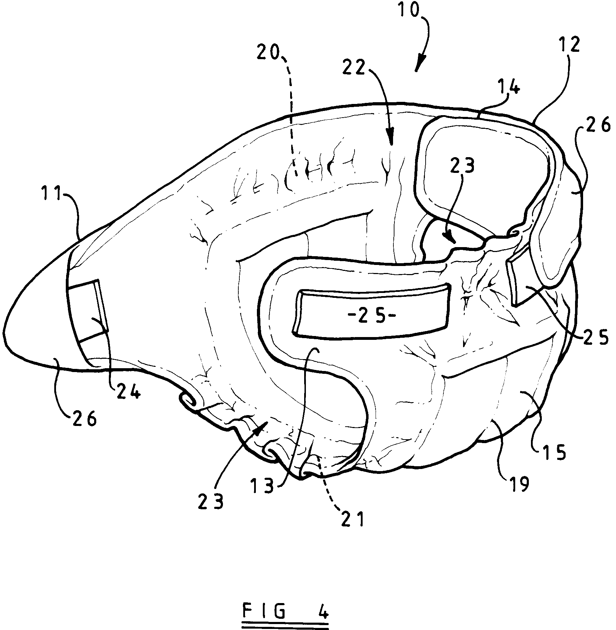

In the preferred form of the present invention this problem can be overcome, or at least reduced, by the provision of pockets 26 at the ends of the tabs 11 and 12. The pockets 26 are positioned on the outer surface 17 of the nappy 10, directly behind the press fasteners 24 on the inner surface 16. By inverting the pocket 26, as is best shown in Figure 4, so that it extends inside out over the inner surface 16, the fastener 24 is concealed inside the pocket 26, and is protected against contact with other materials.

The fasteners 25, bearing loops rather than hooks, do not generally require such protection because they do not have the same tendency to catch on other fabrics or materials.

It should be appreciated that the positions of the fasteners 24 and 25 could be interchanged, although the arrangement illustrated is found preferable at present. The fasteners 25 are preferably relatively long, to allow the fasteners 24 to be attached to them at a range of different positions and thereby accommodate a range of different sizes of baby. A single long fastening strip 25 could be provided across the whole of one end of the nappy 10, comprising the front in normal use, but it is preferred at present to include at least one short elasticated portion 20 on that edge.

Other types of fasteners, such as buttons, press studs or clips might be used instead of press fastener strips as described above. Similarly, variations in the shape or arrangement of the nappy illustrated might also be made. In particular, the nappy might be arranged to partially disassemble or unfold to better facilitate washing and/or drying. In practice it has been found that a nappy as described above does not take significantly longer to machine wash or dry than other clothing of substantial weight. It will be appreciated that the pad 15 could be arranged to unfasten and/or unroll to reduce its thickness and thereby speed washing or drying. This would however increase the complexity and expense of the article, and might consequently be seen as less preferable.

The fasteners 24 could be mounted on the pockets 26, rather than directly behind them, and be concealed inside by the same inverting action. In this case the pockets 26 (and fasteners 24) would be mounted on the inner surface 16, while the fasteners 25 are mounted on the outer surface 17.

Suitable materials for the outer layers and fibrous pad of the nappy are as follows:

Outer layers: 100% twill weave cotton with back side raised.

Fibrous pad: Needle-punched felt comprising the following materials blended and carded in the proportions indicated.

```
Viscose stable fibre CD 3 dr x 51 mm      45%

Polypropylene fibre (100%) 3.3 x 60 mm   24%

Polyester staple fibre SW-233 x 75 mm    31%
(semi-dull raw white non-siliconised)
```

## Claims

1. A washable nappy comprising two sheets (16, 17) of fabric sewn together with a pad (15) of fibrous material sandwiched between them, and provided with reusable fastening elements (24, 25).

2. A nappy according to Claim 1, characterised in that the nappy is of generally oblong shape and has four corners extended to provide fastening tabs (11,12,13,14), the reusable fastening elements (24, 25) being provided on said tabs.

3. A nappy according to Claim 1 or Claim 2, characterised in that said fastening elements comprise complementary strips (24, 25) of press fastening material.

4. A nappy according to Claim 3, characterised in that said complementary strips (24, 25) of press fastening material comprise hook-and-loop material.

5. A nappy according to Claim 4, characterised in that each strip of hook material (24) is mounted on a tab (11, 12) projecting outwardly from the general body of the nappy, said tab being formed of flexible material and provided with a pocket (26) thereon, said strip of hook material (24) being mounted on an outer wall of said pocket, whereby said pocket may be inverted so that the strip lies within the pocket.

6. A nappy according to any of Claims 1 to 5, characterised in that one or both of said two sheets (16, 17) of fabric comprise woven fabric.

7. A nappy according to Claim 6, characterised in that said sheets (16, 17) are formed at least in part from cotton.

8. A nappy according to any of Claims 1 to 7, characterised in that a number of elastic elements (20, 21) are fastened to the nappy and adapted to bunch or gather portions of the nappy in regions between said fastening elements.

9. A nappy according to any of Claims 1 to 8, characterised in that said sheets (16, 17) are sewn together around and across said pad (15) of fibrous material.

FIG 1

FIG 2

<u>F I G   3</u>

FIG 4

**European Patent Office**

# EUROPEAN SEARCH REPORT

Application Number

EP    91 30 8212

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 568 342 (C.W. DAVIS)<br>* the whole document *<br>--- | 1-4,6-9 | A61F13/62 |
| X | US-A-4 402 690 (R. REDFERN)<br>* the whole document *<br>--- | 1-4,6-9 | |
| X | US-A-4 773 906 (S. KRUSHEL)<br>* the whole document *<br>--- | 1-4,6-9 | |
| X<br>A | US-A-4 937 887 (D.N. SCHREINER)<br><br>--- | 1-4,6-9<br>5 | |
| X | WO-A-8 303 754 (F. FREDERICA)<br>* page 12, paragraph 1 – page 14, paragraph 3; figures 1-8 *<br>--- | 5 | |
| X | WO-A-9 007 313 (WORLWIDE BABY BASICS INFANT WEAR LTD)<br>* figures 15-16B * | 1,5 | |
| | ----- | | **TECHNICAL FIELDS SEARCHED (Int. Cl.5)**<br><br>A61F |

The present search report has been drawn up for all claims

| Place of search | Date of completion of the search | Examiner |
|---|---|---|
| THE HAGUE | 15 NOVEMBER 1991 | M. VANMOL |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)